# EUROPÄISCHE PATENTSCHRIFT

(11) **EP 1 729 691 B1**
(45) Veröffentlichungstag und Bekanntmachung des Hinweises auf die Patenterteilung: **05.05.2010**
(21) Anmeldenummer: 04725265.5
(22) Anmeldetag: 02.04.2004
(51) Int. Cl.: A61F 2/44

(54) **BANDSCHEIBENPROTHESE ODER KÜNSTLICHER WIRBELKÖRPER**
INTERVERTEBRAL DISC PROSTHESIS OR ARTIFICIAL VERTEBRAL BODY
PROTHESE DE DISQUE INTERVERTEBRAL OU VERTEBRE ARTIFICIELLE

(43) Veröffentlichungstag der Anmeldung: 13.12.2006
(73) Patentinhaber: Synthes GmbH, 4436 Oberdorf (CH)
(72) Erfinder: STUDER, Armin, CH-6330 Cham (CH); GAGO, Mario, CH-4500 Solothurn (CH); TRACHSEL, Jason, CH-2503 Biel (CH)
(74) Vertreter: Lusuardi, Werther
(86) Internationale Anmeldenummer: PCT/CH2004/000210
(87) Internationale Veröffentlichungsnummer: WO 2005/094733

(56) Entgegenhaltungen:
- EP-A- 0 538 183
- WO-A-00/35385
- WO-A-03/090650
- WO-A-2004/016217
- DD-A- 239 523
- DE-A- 10 132 588
- FR-A- 2 734 148
- US-A- 4 932 975
- US-A1- 2004 010 316

## Beschreibung

Die Erfindung bezieht sich auf eine Bandscheibenprothese oder einen künstlichen Wirbelkörper gemäss dem Oberbegriff des Patentanspruchs 1.
Aus der DE 101 32 588 FEHLING ist eine gattungsgemässe Bandscheibenprothese bekannt, welche zwei Appositionsplatten mit einem dazwischen liegenden Druckfederelement umfassen. Um das Druckfederelement ist ein hohlzylindrischer Mantel angeordnet. Das Druckfederelement besteht aus einer Memory-Metalllegierung, die bei Körpertemperatur superelastische Eigenschaften aufweist, oder in einer anderen Ausführungsform aus einer Tellerfedersäule, welche aus Tellerfedern mit gleicher Steifigkeit zusammengesetzt ist. Nachteilig an dieser bekannten Bandscheibenprothese ist die lineare Federkennlinie des Druckfederelementes, so dass bei einem Druckfederelement, welches auch Stosskräfte aufnehmen soll, insbesondere bei kleinen Druckkräften eine zu geringe Nachgiebigkeit des Druckfederelementes herrscht und daher die Bewegungsfreiheit der Wirbelsäule in diesem Bereich eingeschränkt wird.
Aus der WO 2004/16217 die als nächster Stand der Technik betrachtet wird, und der WO00/35385 sind zwei weitere Bandscheibenprothesen ähnlicher Bauart bekannt, welche jedoch keine Torsionskräfte aufnehmen können.
Hier will die Erfindung Abhilfe schaffen. Der Erfindung liegt die Aufgabe zugrunde, eine Bandscheibenprothese, bzw. einen künstlichen Wirbelkörper zu schaffen, welcher ein aus handelsüblichen Elementen zusammenfügbares, elastisches, eine progressive Federkennlinie aufweisendes Mittelteil mit einer koaxial angeordnete Feder umfasst, welche sowohl als Zugfeder als auch als Druckfeder wirksam ist. Dadurch kann die Schraubenfeder auch Torsionskräfte aufnehmen.

Die Erfindung löst die gestellte Aufgabe mit einer Bandscheibenprothese oder einem künstlichen Wirbelkörper, welche die Merkmale des Anspruchs 1 aufweisen.

Die durch die Erfindung erreichten Vorteile sind im wesentlichen darin zu sehen, dass dank der erfindungsgemässen Bandscheibenprothese oder des künstlichen Wirbelkörpers
- bei kleinen Druckkräften eine ausreichend grosse elastische Nachgiebigkeit und Dämpfung gewährleistet ist. Dadurch wird eine ausreichende Bewegungsfreiheit der Wirbelsäule in diesem Bereich gewährleistet; und
- bei hohen Druckbelastungen oder Stossbelastungen keine grossen Federwege zur Aufnahme der Druckkraft notwendig sind; und
- der Steifigkeitssprung zwischen einem gesunden Wirbelsegment und dem mit einer Prothese versehenen Wirbelsegment kann harmonisch verringert werden, so dass ein Verhalten erzielt wird, wie bei einer gesunden Bandscheibe.

Bei einer bevorzugten Ausführungsform umfassen die elastischen Mittel mindestens zwei koaxial angeordnete Tellerfedern.

Bei einer besonderen Ausführungsform ist eine Zentrierung für die elastischen Mittel vorgesehen. Durch die Zentrierung können die Tellerfedern nicht verrutschen und werden in ihrer Position gehalten. Zudem können durch die Zentrierung die aneinanderliegenden Tellerfedern sehr grosse Kräfte aufnehmen und als Sicherheit dienen.

Bei einer weiteren Ausführungsform ist die Zentrierung flexibel ausgestaltet, so dass sie eine axiale Führung innerhalb eines Kegelbereiches gestattet. Der dadurch erzielbare Vorteil liegt darin, dass die Bandscheibenprothese nicht nur koaxial elastisch ausgebildet ist; die Appositionsplatten können auch schräg oder rotativ zueinander bewegt werden.

Bei einer weiteren Ausführungsform weisen die Tellerfedern zentrale Bohrungen auf und die Zentrierung ist als innerer, durch diese Bohrungen durchgeführter Faltenbalg ausgestaltet.
Bei einer weiteren Ausführungsform weisen mindestens zwei Tellerfedern eine unterschiedliche Steifigkeit auf. Dadurch kann die progressive Federkennlinie allein durch die aus Tellerfedern bestehende Feder hergestellt werden. Ein weiterer Vorteil liegt in der grossen Auflagefläche, was zu einer kleinen Flächenpressung und einem geringen Abrieb führt.
Bei einer weiteren Ausführungsform sind die Tellerfedern zu Tellerfederpaketen mit mehreren gleichgerichtet geschichteten Tellerfedern zusammengefügt. Dies hat den Vorteil, dass die progressive Federkennlinie nur durch die aus Tellerfedern bestehende Feder herstellbar ist. Bei einer Kombination mit einem Faltenbalg ergibt sich ein zusätzlicher Vorteil, indem die Federkennlinie zusätzlich beinflussbar ist.

Bei einer weiteren Ausführungsform liegen, bis zu einem axialen Federweg X ≠ 0, nur die Federn an beiden Appositionsplatten an. Der erzielbare Vorteil liegt in der progressiven Federkennlinie. Die zweite Feder kann auch als Zentrierung für die Tellerfedern dienen. Weitere Vorteile liegen darin begründet, dass allfällige Abriebpartikel nicht in den Körper des Patienten entweichen können. Diese Ausführungsform erlaubt auch eine Extensions/Flexions-Bewegung mit niedriger Steifigkeit und gibt einen Schutz gegen das Einwachsen von Bindegewebe.
Bei einer weiteren Ausführungsform weist das Mittelteil aussen einen aus einem Faltenbalg bestehenden Mantel auf. Dieser Mantel gibt Schutz vor dem Eindringen von Körperflüssigkeit.
Bei einer weiteren Ausführungsform ist das Mittelteil mittels eines Schnappverschlusses mit den zwei Appositionsplatten lösbar verbindbar.
Bei einer weiteren Ausführungsform ist sind die Appositionsplatten über Gleitlager, welche vorzugsweise aus einem keramischen Material bestehen, mit dem Mittelteil verbunden. Die Gleitlager sind vorteilhafterweise derart ausgebildet sind, dass die Appositionsplatten gegenüber dem Mittelteil eine beschränkte Translation quer zur Längsachse ausführen können.
Die Translation beträgt vorzugsweise +/- 0,5 mm. Somit sind über die Gleitlager und über die Aufnahmen der Tellerfedern sowie über die mögliche Rotation von ca. +/- 30° um die Längsachse der Prothese alle 6 Freiheitsgrade abgedeckt.

Bei einer weiteren Ausführungsform weisen die Tellerfedern einen Anschlag auf, welcher ihre Kompressibilität beschränkt. Der Anschlag schützt die Tellerfedern vor eine Überbeanspruchung. Dadurch können auch höhere Kräfte aufgenommen werden, ohne dabei die Tellerfedern zu überlasten.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 einen medio-lateralen Schnitt durch eine Ausführungsform der erfindungsgemässen Bandscheibenprothese; und
Fig. 2 einen medio-lateralen Schnitt durch eine weitere Ausführungsform der erfindungsgemässen Bandscheibenprothese; und

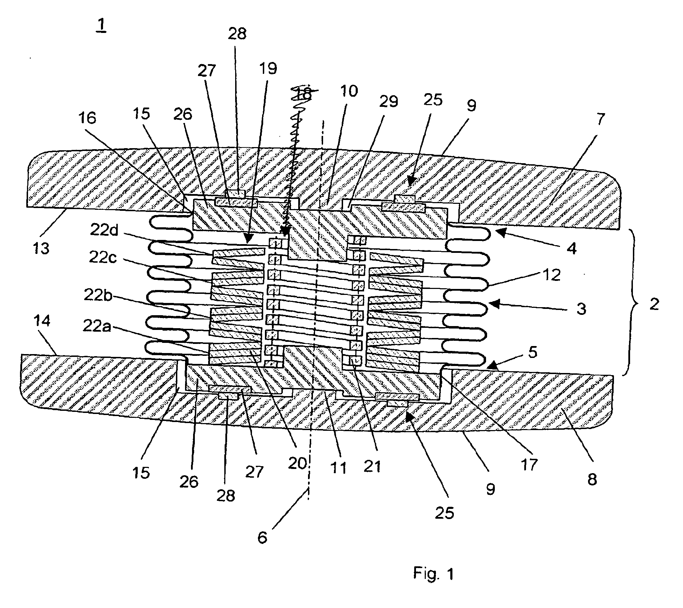

Die in Fig. 1 dargestellte Ausführungsform der Bandscheibenprothese 1 umfasst im wesentlichen ein hohlzylindrisches, elastisches Mittelteil 1 mit einem als Faltenbalg 12 ausgebildeten Mantel 3, einem oberen Ende 4, einem unteren Ende 5 und einer zentralen Längsachse 6, sowie eine am oberen Ende 4 des Mittelteils 1 quer zur Längsachse 5 angeordnete, obere Appositionsplatte 7, welche zur Anlage an die Grundplatte eines Wirbelkörpers geeignet ist, und eine am unteren Ende 5 des Mittelteils 1 quer zur Längsachse 6 angeordnete, untere Appositionsplatte 8 welche zur Anlage an die Deckplatte eines Wirbelkörpers geeignet ist. Die beiden Appositionsplatten 7;8 haben gegen Aussen eine konvex gewölbte Oberfläche 9 und weisen nach innen gerichtete axiale Zapfen 10 und 11 auf. Der Mantel 3 besteht bei dieser Ausführungsform aus einem Faltenbalg 12, welche an den beiden Appositionsplatten 7;8 befestigt ist. Je nach Material kann der Faltenbalg 12 an den Appositionsplatten 7;8 angeschweisst, verstemmt oder eingepresst sein.

Die obere Appositionsplatte 7 weist eine gegen das Mittelteil 2 gerichtete untere Oberfläche 13 auf und die untere Appositionsplatte 8 weist analog eine gegen das Mittelteil 2 gerichtete obere Oberfläche 14 auf. Die untere und die obere Oberfläche 13,14 sind hier mit je einer kreisringartig angeordneten Nute 15 zur Aufnahme der an den Enden 16;17 des Faltenbalges 12 befestigten Deckplatten 26 versehen. Die Zapfen 10;11 ragen koaxial zur Längsachse 6 in zur Längsachse 6 konzentrische Vertiefungen 29 an den aussenstehenden Oberflächen der Deckplatten 26. Damit die Appositionsplatten 7;8 relativ zum Mittelteil 2 lateral verschiebbar sind, weisen die Vertiefungen 29 quer zur Längsachse 6 einen grösseren Durchmesser auf als die darin bewegbaren Zapfen 10;10 an den Appositionsplatten 10; 11. In den kreisringförmigen Nuten 15 in der unteren und oberen Oberfläche 13;14 der Appositionsplatten 7;8 sind zur Längsachse 6 konzentrisch die äusseren Ringelemente 28 eingelassen, welche an den in die aussenstehenden Oberflächen der Deckplatten 26 eingefügten inneren Ringelementen 27 aufliegen. Die paarweise aufeinander aufliegenden inneren und äusseren Ringelemente 27;28 bilden je ein Gleitlager 25, derart, dass die Appositionsplatten 7;8 lateral innerhalb des durch die Zapfen 10;11 und die Vertiefungen 29 zugelassenen Spiels relativ zum Mittelteil 2 verschiebbar sind.

Um die Schraubenfeder 21 herum sind die elastischen Mittel 19 angeordnet, welche aus Tellerfedern 20 zusammengefügt sind. In der hier dargestellten Ausführungsform steht im unbelasteten Zustand der Bandscheibenprothese 1 nur die Schraubenfeder 21 im Kontakt mit den zwei Deckplatten 26, so dass bei einer Belastung der Bandscheibenprothese zuerst nur die Schraubenfeder 21 zusammengepresst wird. Nach Zurücklegen eines Federweges s = X kommen auch die elastischen Mittel 19 mit beiden Appositionsplatten 7;8 in Berührung, so dass bei einer weiteren Verschiebung der zwei Appositionsplatten 7;8 gegeneinander s > X eine höhere Federrate wirksam wird. In der hier dargestellten Ausführungsform sind die elastischen Mittel 19 aus Tellerfederpaketen 22a;22b;22c;22d mit gleichen Tellerfedern 20 zusammengesetzt, wobei jedoch das erste Tellerfederpaket 22a aus einer drei gleichgerichtet geschichtete Tellerfedern 20 umfassenden Tellefedergruppe besteht, das zweite und dritte Tellerfederpaket 22b;22c je zwei gegeneinander gerichtete Gruppen aus je zwei gleichgerichtet geschichteten Tellerfedern 20 besteht und das vierte Tellerfederpaket 22d aus zwei gegeneinander gerichteten Tellerfedern 20 besteht. Durch diese Ausgestaltung der Tellerfederpakete 22a;22b;22c;22d ist eine progressive Federkennlinie der elastischen Mittel 19 erreichbar.

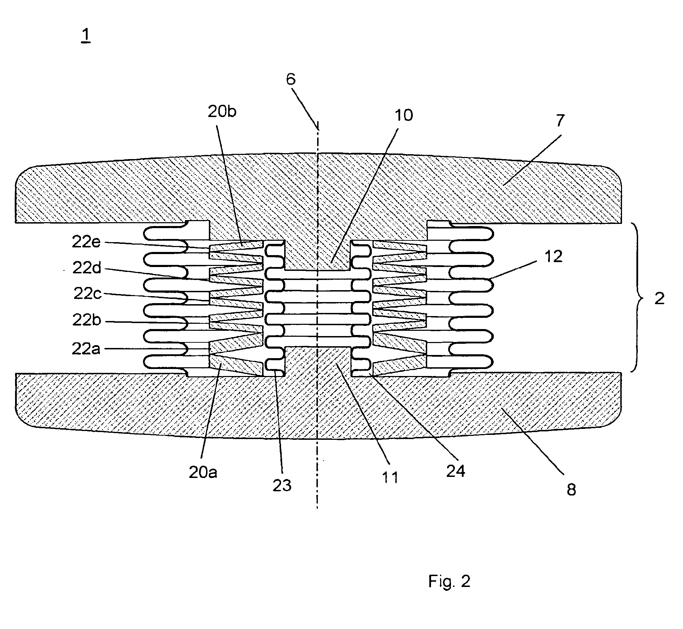

Die in Fig. 2 dargestellte Ausführungsform der Bandscheibenprothese 1 unterscheidet sich von der in Fig. 1 dargestellten Ausführungsform nur darin, dass erstens anstelle der Feder 21 ein innerer Faltenbalg 23 zwischen den Appositionsplatten 7;8 angeordnet ist, zweitens die elastischen Mittel 19 aus einem ersten Tellerfederpaket 22a mit ersten Tellerfedern 20a und vier weiteren Tellerfederpaketen 22b;22c;22d;22e mit zweiten Tellerfedern 20b bestehen und drittens das Mittelteil 2 ohne Deckplatten 26 (Fig. 1) mit den Appositionsplatten 7;8 verbunden ist. Dabei weisen die ersten Tellerfedern 20a eine höhere Steifigkeit auf als die zweiten Tellerfedern 20b, so dass die elastischen Mittel 19 eine progressive Federkennlinie aufweist. Der innere Faltenbalg 23 wird parallel zur Längsachse 6 durch die Bohrungen 24 der Tellerfedern 20 durchgeführt und dient als Zentrierung für die Tellerfedern 20. Der innere Faltenbalg 23 ist axial und bezüglich Biegung flexibel, so dass die Appositionsplatten 7;8 auch schräg gegeneinander bewegt werden können und die Längsachse 6 innerhalb eines Kegelbereichs gekrümmt werden kann.

## Patentansprüche

1. Bandscheibenprothese (1) oder Zwischenwirbelimplantat umfassend:
A) ein Mittelteil (2) mit einer zentralen Längsachse (6), einem oberen Ende (4) und einem unteren Ende (5);
B) eine am oberen Ende (4) des Mittelteils (2) quer zur Längsachse (6) angeordnete, obere Appositionsplatte (7), welche zur Anlage an die Grundplatte eines Wirbelkörpers geeignet ist;
C) eine am unteren Ende (5) des Mittelteils (2) quer zur Längsachse (6) angeordnete, untere Appositionsplatte (8), welche zur Anlage an die Deckplatte eines Wirbelkörpers geeignet ist; wobei
D) das Mittelteil (2) elastische Mittel (19) umfasst;
E) die elastischen Mittel (19) eine progressive Federkennlinie aufweisen; und dass
F) eine Zentrierung für die elastischen Mittel (19) vorgesehen ist,
**dadurch gekennzeichnet, dass**
G) das Mittelteil (2) eine koaxial angeordnete Feder (21) umfasst, welche sowohl als Zugfeder als auch als Druckfeder wirksam ist.

2. Bandscheibenprothese (1) nach Anspruch 1, **dadurch gekennzeichnet, dass** die elastischen Mittel (19) mindestens zwei koaxial angeordnete Tellerfedern (20) umfassen.

3. Bandscheibenprothese (1) nach Anspruch 2, **dadurch gekennzeichnet, dass** mindestens zwei der Tellerfedern (20) eine unterschiedliche Steifigkeit aufweisen.

4. Bandscheibenprothese (1) nach Anspruch 2 oder 3, **dadurch gekennzeichnet, dass** die Tellerfedern (20) zu Tellerfederpaketen (22) mit mehreren gleichgerichtet geschichteten Tellerfedern (20) zusammengefügt sind.

5. Bandscheibenprothese (1) nach einem der Ansprüche 1 bis 4, **dadurch gekennzeichnet, dass** das Mittelteil (2) hohlkörperartig ausgebildet ist.

6. Bandscheibenprothese (1) nach einem der Ansprüche 1 bis 5, **dadurch gekennzeichnet, dass** die Zentrierung flexibel ausgestaltet ist und eine axiale Führung innerhalb eines Kegelbereiches gestattet.

7. Bandscheibenprothese (1) nach einem der Ansprüche 1 bis 6, **dadurch gekennzeichnet, dass** die Tellerfedern (20) zentrale Bohrungen (24) aufweisen und dass die Zentrierung als innerer, durch diese Bohrungen (24) durchgeführter Faltenbalg ausgestaltet ist.

8. Bandscheibenprothese (1) nach einem der Ansprüche 1 bis 7, **dadurch gekennzeichnet, dass** bis zu einem axialen Federweg X ≠ 0 nur die Federn (21) an beiden Appositionsplatten (7;8) anliegen.

9. Bandscheibenprothese (1) nach einem der Ansprüche 1 bis 8, **dadurch gekennzeichnet, dass** das Mittelteil (2) aussen einen aus einem Faltenbalg (12) bestehenden Mantel (3) aufweist.

10. Bandscheibenprothese (1) nach einem der Ansprüche 1 bis 9, **dadurch gekennzeichnet, dass** das Mittelteil (2) mittels eines Schnappverschlusses mit den zwei Appositionsplatten (7;8) lösbar verbindbar ist.

11. Bandscheibenprothese (1) nach einem der Ansprüche 1 bis 10, **dadurch gekennzeichnet, dass** die Appositionsplatten (7,8) über Gleitlager (25), welche vorzugsweise aus einem keramischen Material bestehen, mit dem Mittelteil (2) verbunden sind.

12. Bandscheibenprothese (1) nach Anspruch 11, **dadurch gekennzeichnet, dass** das die Gleitlager (25) derart ausgebildet sind, dass die Appositionsplatten (7,8) gegenüber dem Mittelteil (2) eine beschränkte Translation quer zur Längsachse (6) ausführen können.

13. Bandscheibenprothese (1) nach einem der Ansprüche 2 bis 12, **dadurch gekennzeichnet, dass** die Tellerfedern (20) einen Anschlag aufweisen, welcher ihre Kompressibilität beschränkt.

## Claims

1. Prosthetic intervertebral disc (1) or intervertebral implant, comprising:
A) a middle part (2) having a central longitudinal axis (6), an upper end (4) and a lower end (5);
B) an upper apposition plate (7), which is arranged across the longitudinal axis (6) on the upper end (4) of the middle part (2) and is suitable for coming in contact with the lower plate of a vertebra;
C) a lower apposition plate (8) which is arranged across the longitudinal axis (6) on the lower end (5) of the middle part (2) and is suitable for coming in contact with the upper plate of a vertebra; and whereby
D) the middle part (2) comprises elastic means (19);
E) the elastic means (19) have a progressive spring characteristic; and
F) a centering is provided for the elastic means (19),
**characterized in that**
G) the middle part (2) comprises a spring (21) arranged coaxially, acting as both a tension spring and a compression spring.

2. Prosthetic intervertebral disc (1) according to Claim 1, **characterized in that** the elastic means (19) comprise at least two cup springs (20) arranged coaxially.

3. Prosthetic intervertebral disc (1) according to Claim 2, **characterized in that** at least two of the cup springs (20) have a different stiffness.

4. Prosthetic intervertebral disc (1) according to Claim 2 or 3, **characterized in that** the cup springs (20) are joined together to form cup spring assemblies (22) having a plurality of cup springs (20) that are stacked together and point in the same direction.

5. Prosthetic intervertebral disc (1) according to any one of Claims 1 through 4, **characterized in that** the middle part (2) is designed like a hollow body.

6. Prosthetic intervertebral disc (1) according to any one of Claims 1 through 5, **characterized in that** the centering is designed to be flexible and allows axial guidance within a conical area.

7. Prosthetic intervertebral disc (1) according to any one of Claims 1 through 6, **characterized in that** the cup springs (20) have central bores (24) and the centering is designed as internal bellows passing through these bores (24).

8. Prosthetic intervertebral disc (1) according to any one of Claims 1 through 7, **characterized in that** up to an axial spring deflection X ≠ 0, only the springs (21) are in contact with both apposition plates (7; 8).

9. Prosthetic intervertebral disk (1) according to any one of Claims 1 through 8, **characterized in that** the middle part (2) has a jacket (3) consisting of bellows (12) on the outside.

10. Prosthetic intervertebral disc (1) according to any one of Claims 1 through 9, **characterized in that** the middle part (2) is detachably connectable to the two apposition plates (7; 8) by means of a snap closure.

11. Prosthetic intervertebral disc (1) according to any one of Claims 1 through 10, **characterized in that** the apposition plates (7; 8) are connected to the middle part (2) via friction bearings (25), which are preferably made of a ceramic material.

12. Prosthetic intervertebral disc (1) according to Claim 11, **characterized in that** the friction bearings (25) are designed so that the apposition plates (7; 8) can execute a limited translational motion across the longitudinal axis (6) with respect to the middle part (2).

13. Prosthetic intervertebral disc (1) according to any one of Claims 2 through 12, **characterized in that** the cup springs (20) have a stop which limits their compressibility.

## Revendications

1. Prothèse de disque intervertébral (1) ou implant intervertébral comprenant :
A) un élément central (2) présentant un axe longitudinal central (6), une extrémité supérieure (4) et une extrémité inférieure (5) ;
B) une plaque d'apposition supérieure (7) disposée transversalement à l'axe longitudinal (6) au niveau de l'extrémité supérieure (4) de l'élément central (2), laquelle plaque est appropriée pour venir en contact avec le plateau inférieur d'un corps vertébral ;
B) une plaque d'apposition inférieure (8) disposée transversalement à l'axe longitudinal (6) au niveau de l'extrémité inférieure (5) de l'élément central (2), laquelle plaque est appropriée pour venir en contact avec le plateau supérieur d'un corps vertébral ; dans laquelle
D) l'élément central (2) comprend des moyens élastiques (19) ;
E) les moyens élastiques (19) présentent une caractéristique du ressort progressive ; et en ce que
F) un centrage est prévu pour les moyens élastiques (19),
**caractérisée en ce que**
G) l'élément central (2) comprend un ressort coaxial (21), lequel ressort agit aussi bien en tant que ressort de rappel qu'en tant que ressort de pression.

2. Prothèse de disque intervertébral (1) selon la revendication 1, **caractérisée en ce que** les moyens élastiques (19) comprennent au moins deux ressorts à disques (20) coaxiaux.

3. Prothèse de disque intervertébral (1) selon la revendication 2, **caractérisée en ce qu'**au moins deux des ressorts à disques (20) présentent une rigidité différente.

4. Prothèse de disque intervertébral (1) selon la revendication 2 ou 3, **caractérisée en ce que** les ressorts à disques (20) sont assemblés pour former des paquets de ressorts à disques (22) comprenant plusieurs ressorts à disques (20) superposés, dirigés dans le même sens.

5. Prothèse de disque intervertébral (1) selon l'une quelconque des revendications 1 à 4, **caractérisée en ce que** l'élément central (2) est réalisé sous la forme d'un corps creux.

6. Prothèse de disque intervertébral (1) selon l'une quelconque des revendications 1 à 5, **caractérisée en ce que** le centrage est réalisé de manière flexible et permet un guidage axial à l'intérieur d'une zone conique.

7. Prothèse de disque intervertébral (1) selon l'une quelconque des revendications 1 à 6, **caractérisée en ce que** les ressorts à disques (20) présentent des alésages centraux (24) et **en ce que** le centrage est réalisé sous la forme d'un soufflet interne passant à travers ces alésages (24).

8. Prothèse de disque intervertébral (1) selon l'une quelconque des revendications 1 à 7, **caractérisée en ce que**, jusqu'à un allongement du ressort axial X ≠ 0, seuls les ressorts (21) sont en contact avec les deux plaques d'apposition (7 ; 8).

9. Prothèse de disque intervertébral (1) selon l'une quelconque des revendications 1 à 8, **caractérisée en ce que** l'élément central (2) présente, sur la face externe, une enveloppe (3) formée par un soufflet (12).

10. Prothèse de disque intervertébral (1) selon l'une quelconque des revendications 1 à 9, **caractérisée en ce qu'**une fermeture à encliquetage permet de relier de manière amovible l'élément central (2) aux deux plaques d'apposition (7 ; 8).

11. Prothèse de disque intervertébral (1) selon l'une quelconque des revendications 1 à 10, **caractérisée en ce que** les plaques d'apposition (7, 8) sont reliées à l'élément central (2) par l'intermédiaire de paliers lisses (25) faits de préférence d'une matière céramique.

12. Prothèse de disque intervertébral (1) selon la revendication 11, **caractérisée en ce que** les paliers lisses (25) sont réalisés de telle sorte que les plaques d'apposition (7, 8) ne puissent effectuer, par rapport à l'élément central (2), qu'une translation limitée transversalement à l'axe longitudinal (6).

13. Prothèse de disque intervertébral (1) selon l'une quelconque des revendications 2 à 12, **caractérisée en ce que** les ressorts à disques (20) présentent une butée, laquelle limite leur compressibilité.
